# EUROPEAN PATENT APPLICATION

(11) **EP 1 837 038 A1**
(43) Date of publication of application: **26.09.2007**
(21) Application number: 06270032.3
(22) Date of filing: 20.03.2006
(51) Int. Cl.: A61L 24/00, A61L 24/04

(54) **Tissue-Adhesive Formulations**

(71) Applicant: Tissuemed Limited, Leeds LS14 6UF (GB)
(72) Inventor: Kettlewell, Graeme, Leeds, LS14 6UF (GB); Mandley, David, Leeds, LS14 6UF (GB); Fortune, David, Leeds, LS14 6UF (GB); Thompson, Ian, Leeds, LS14 6UF (GB); Morris, Diane, Leeds, LS14 6UF (GB)
(74) Representative: Jones, Stephen Anthony

(57) **Abstract**

A tissue-adhesive formulation comprises a particulate material having tissue-reactive functional groups, in admixture with a particulate buffer material. The formulation is preferably free or substantially free of materials of human or animal origin, and may consist, or consist essentially of, an anhydrous or partially hydrated blend of particulate material having tissue-reactive functional groups and particulate buffer material. The formulation is preferably buffered to a pH in the range 8-12, and the buffer material may comprise carbonate, phosphate, HEPES or borate buffers. There is also disclosed a sheet comprising a structural support coated on at least one side thereof with such a formulation. The formulation and sheet are useful for joining a tissue surface to another tissue, or for sealing a tissue surface.

## Description

### Field of the Invention

This invention relates to materials suitable for use as tissue adhesives and sealants, and to a flexible multilamellar sheet, patch or film comprising such materials for topical application to internal and external surfaces of the body, for therapeutic purposes. The invention also relates to a process for the preparation of such products, and to methods of using such products. In particular the invention relates to materials that are formulated as loose or compacted powders and to a self-adhesive, biocompatible and hydratable polymeric sheet with such materials applied to a suitable support, which may be used for therapeutic purposes such as wound healing, joining, sealing and reinforcing weakened tissue, and for drug delivery, and to a process for preparing, and methods of using, such a sheet.

### Background of the Invention

There is considerable interest in the use, for a number of surgical or other therapeutic applications, of materials that adhere to biological tissues eg as an alternative to the use of mechanical fasteners such as sutures, staples etc. Formulations of such materials that have hitherto been proposed include viscous solutions or gels that are either manufactured in that form or are prepared immediately prior to use by mixing of the ingredients. Such formulations are then applied to the tissue surface using a suitable applicator device such as a syringe.

Formulations of the type described above suffer from a number of disadvantages. If the formulation is of low viscosity, it may spread from the area of application and hence be difficult to apply precisely to the desired area of tissue. If the formulation is more viscous, on the other hand, it may be difficult to dispense. In either case, the formulation, being prepared in hydrated form, may have a limited lifetime and may be subject to premature curing. It may therefore be necessary for the whole of the formulation to be used at once or discarded. Also, the preparation of formulations immediately prior to use by mixing of ingredients is obviously laborious and time-consuming. In addition to these drawbacks, the degree of adhesion between tissue surfaces that is provided by such formulations may be less than would be desired.

Formulations of tissue adhesive materials have also been applied to a suitable support for application to the tissue surface. The use of therapeutic materials in the form of a sheet, patch or film, for topical administration to either internal or external organs of the body, is well documented for a wide range of medical applications. A disadvantage of products proposed hitherto, however, is that the degree of adhesion to the underlying tissue, particularly in the longer term, may be inadequate. While the initial adhesion may be satisfactory, the sheet may subsequently become detached from the tissue, often after only a few seconds or minutes, eg as a result of hydration of the sheet following its application. In addition, the flexibility of the product may be insufficient for it to conform readily to the surface to which it is applied, which may also have an adverse effect on its adhesion.

As a result of the inadequate adhesion of these products, it may be necessary to provide further reinforcement, eg through mechanical attachment using sutures, staples or the like. Alternatively, energy (eg light or heat energy) may be applied in order to initiate chemical bonding of the adhesive formulation to the underlying tissue, and hence bonding of the tissue surfaces to each other. Clearly, such approaches introduce further drawbacks. The use of mechanical fastenings such as sutures or staples is often the very thing that the use of such products is intended to replace or avoid. In many instances, the use of such fastenings is either not wholly effective (eg on the lung) or undesirable, as their introduction gives rise to further areas of tissue weakness. The use of external energy requires the provision and operation of a source of such energy. Such energy sources may be expensive and difficult to operate, particularly in the confines of an operating theatre or similar environment. Also, the use of external energy for attachment can be both time-consuming and (in some cases) requires significant careful judgement on the part of the surgeon, to evaluate when sufficient energy has been delivered to effect attachment without damaging the underlying tissue.

WO-A-2004/087227 describes tissue-adhesive formulations comprising an admixture of particulate material containing tissue-reactive groups and particulate cross-linkable material. The former may be a synthetic polymer containing groups such as N-hydroxysuccinimide ester groups, and the latter may be a protein, human serum albumin being the material that is specifically described. Formulations of this type have been found to be effective, but may not be ideal in several respects. For instance, the biodegradation of the product following use may take longer than might be desired for certain applications. Also, there is a perceived risk associated with the use of materials of human or animal original, such as human serum albumin, due to the risk (albeit theoretical) of transmission of pathogenic agents such as viruses and prion proteins.

There have now been devised improved formulations of tissue-adhesive materials and sheets or the like of the general type described above that overcome or substantially mitigate the above-mentioned and/or other disadvantages of the prior art. ln particular, it has surprisingly been found that formulations of particulate material provide satisfactory results when the cross-linkable material that constitutes a substantial part of the formulations described in WO-A-2004/087227 is not present, and that such formulations offer certain advantages.

### Brief Summary of the Invention

According to a first aspect of the invention, there is provided a tissue-adhesive formulation comprising a particulate material having tissue-reactive functional groups, in admixture with a particulate buffer material.

The formulation according to the invention is advantageous primarily in that it is of very simple form, and hence can be prepared relatively easily and reproducibly, with good homogeneity that leads to consistent performance. In addition, the formulation may have particularly beneficial biodegradation characteristics and/or improved biocompatibility due to the presence, generally, of only one major component in the formulation. As the formulation generally contains no material of human or animal origin, the theoretical risk associated with the use of such materials is eliminated.

In addition, the formulation can be easily applied to a tissue surface using a simple applicator or delivery device. As it is applied in solid form, the particulate formulation adheres to the tissue surface and does not spread unduly. The formulation exhibits good initial adhesion to the tissue surface, this being believed to be due to van der Waals forces and/or hydrogen bonding between the formulation and the tissue surface. On contact with the tissue surface the formulation becomes hydrated, thereby causing reaction between the tissue-reactive functional groups and the underlying tissue surface. Such reactions between the tissue-reactive functional groups and the underlying tissue results in high adhesion between the formulation and the tissue surface, and hence between tissues that are joined using the adhesive formulation. Reaction may also take place between the tissue-reactive functional groups and the other components of the formulation to form a strong, flexible and tissue-adherent gel. This formulation thus has the ability to absorb endogenous amino acid-, peptide-, polypeptide- or protein-rich body fluids (eg blood, plasma, serum and extracellular, interstitial, intracavity or lymph fluids) as a consequence of application onto exuding tissue surfaces, as well as any additional solutions used to hydrate the formulation following application (such fluids can be commonly used solutions used in surgical irrigation), and to cross-link and to adhere and cross-link to cross-linkable molecules on the tissue surface, thereby providing an adhesive, sealant, haemostatic and pneumostatic function.

In addition, because the formulation is made up in solid form that is, until hydrated by contact with the tissue surface (and subsequent hydration), essentially inactive, the formulation is not prone to premature reaction and as a result its shelf-life may be considerable, eg more than six months when stored appropriately at room temperature. This further enables the formulation to be packaged in relatively large quantities that can be dispensed and used over a considerable time period, without the risk of substantial wastage.

In another aspect, the invention provides a tissue-adhesive formulation comprising a particulate material having tissue-reactive functional groups, wherein the formulation is free or substantially free of materials of human or animal origin, eg wherein such materials account for less than 1% w/w, more preferably less than 0.5% w/w or less than 0.1% w/w, of the formulation. One situation in which a relatively small proportion of material of human or animal origin may be present is where that material takes the form of one or more therapeutically active agents that are included in the formulation and are of such origin.

Preferred embodiments of the formulation may also be essentially binary mixtures of the particulate material having tissue-reactive functional groups and the particulate buffer material. Upon application to tissue, the formulation will become hydrated, but it will be appreciated that a certain amount of moisture may be present following manufacture and prior to use. The invention thus provides a tissue-adhesive formulation that consists, or consists essentially of, an anhydrous or partially hydrated blend of particulate material having tissue-reactive functional groups and particulate buffer material.

According to another aspect of the present invention, there is provided a sheet having a multilayer structure, said structure comprising a structural support coated on at least one side thereof with a tissue-adhesive formulation as defined above.

In preferred embodiments of the invention, the tissue-adhesive formulation is applied to the support by mechanically compressing a blend of material containing tissue-reactive functional groups (hereinafter referred to as "tissue-reactive material") and the buffer material, both in particulate form, onto one or both sides of the support.

The sheet according to the invention is advantageous primarily in that it bonds effectively to tissue, enabling it to be used in a variety of medical applications. The invention enables coating of the tissue-reactive materials onto (and into) a three-dimensional structural support, whilst maintaining the pliability and physical properties of the support. Furthermore, the adhesive performance of the tissue-reactive materials is not compromised when delivered to the target tissue in this form. Where, as in preferred embodiments, the support is perforated, the perforations provide a means of anchoring the tissue-reactive materials in the support. This reduces or eliminates cracking and crumbling of the tissue-reactive material as it is applied to the support, which would result in sub-optimal coverage of the target tissue, and thereby compromise the adhesive/sealant effects of the sheet.

The sheet may exhibit good initial adhesion to the tissue to which it is applied (and may thus be described as "self-adhesive"), and furthermore remains well-adhered to the tissue over a longer timescale. Without wishing to be bound by any theory, it is believed that the initial adhesion of the sheet to the tissue is attributable to electronic bonding of the sheet to the tissue, and this is supplemented or replaced by chemical bonding between the tissue-reactive functional groups of the formulation and the tissue, in particular between amine and/or thiol groups on the tissue surface and the tissue-reactive groups of the sheet. Where the structural support of the device is perforated, and is coated on both sides with the tissue-adhesive formulation, the perforations facilitate hydration and cross-linking of the formulation on both sides of the support such that the support becomes enclosed within a three-dimensional matrix of cross-linked material.

The use of the sheet reduces or eliminates the need for additional means of mechanical attachment to the tissue (eg sutures or staples), or the need to provide external energy in the form of heat or light to bring about adherence of the sheet to the underlying tissue. Another advantage of the sheet according to the invention is that it is applied to the tissue as a preformed article, rather than being prepared by mixing of materials immediately prior to use.

By the term "sheet" is meant a three-dimensional article with a thickness that is considerably less than its other dimensions. Such an article may alternatively be described as a patch or a film.

According to another aspect of the invention, there is provided a method for the manufacture of a sheet according to the invention, which method comprises forming a structural support, and coating at least one side of said core with a tissue-adhesive formulation as defined above.

In a yet further aspect, the invention also provides a method of joining a tissue surface to another tissue, or of sealing a tissue surface, which method comprises applying to the tissue surface a formulation or a sheet as defined above.

### Brief Description of the Drawings

Figure 1 is a schematic representation of the reaction between a tissue-reactive functional group (in the illustrated case an N-hydroxysuccinimide ester) and an amine-containing molecule such as a tissue protein.
Figure 2 shows the introduction of carboxyl group-bearing side chains into a poly(vinyl alcohol - vinyl acetate) copolymer.
Figure 3 represents the formation of a poly(N-vinyl-2-pyrrolidone-co-acrylic acid) copolymer.
Figure 4 shows the mechanism of free radical initiation of a polymerisation reaction.
Figure 5 illustrates the synthesis of a tissue reactive material.
Figure 6 is a schematic sectional view of a sheet according to the invention.

### Detailed Description of the Invention

### Abbreviations

- AAc: acrylic acid
- AIBN: azo-iso-butyronitrile
- DCC: dicyclohexylcarbodiimide
- DCU: dicyclohexylurea
- DMF: dimethylformamide
- ENT: ear, nose and throat
- *M*ₙ: number average molecular weight
- *M*_{w}: weight average molecular weight
- NHS: N-hydroxysuccinimide
- PLG: poly(DL-lactide-co-glycolide)
- poly(VP-AAc): copolymer of vinyl pyrrolidone and acrylic acid
- poly(VP-AAc(NHS): copolymer of vinyl pyrrolidone and acrylic acid NHS ester
- poly(VP-AAc-AAc(NHS)): terpolymer of vinyl pyrrolidone, acrylic acid and acrylic acid NHS ester
- PVA: polyvinylalcohol
- PVP: poly(N-vinyl-2-pyrrolidone)

### Composition of the formulation

The formulation according to the first aspect of the invention comprises particulate material comprising tissue-reactive functional groups and particulate buffer material.

These two components may be blended in suitable proportions, which may depend on the particular materials used, as well as the desired properties of the resulting blend. Typically, the ratio by weight of tissue-reactive material to buffer material will be between 90:10 and 99:1, more preferably between 92:8 and 97:3.

The particles that make up the formulation have a wide range of particle sizes. The median particle size may, for instance, lie in the range 5µm to 500µm, more preferably 5µm to 250µm.

### Nature of the tissue-reactive material

The tissue-reactive material is preferably polymeric in nature. Most preferably, the polymer is a synthetic polymer.

By "tissue-reactive functional groups" is meant functional groups capable of reacting with other functional groups present in the tissue surface so as to form covalent bonds between the formulation and the tissue. Tissues generally consist partly of proteins, which commonly contain thiol and primary amine moieties. Many functional groups such as imido ester, p-nitrophenyl carbonate, NHS ester, epoxide, isocyanate, acrylate, vinyl sulfone, orthopyridyl-disulfide, maleimide, aldehyde, iodoacetamide, and others, may react with thiols or primary amines, and therefore constitute "tissue-reactive functional groups". As used herein, the term "NHS" or "NHS ester" is intended to encompass not only N-hydroxysuccinimide itself, but also derivatives thereof in which the succinimidyl ring is substituted. An example of such a derivative is N-hydroxysulfosuccinimidyl and salts thereof, particularly the sodium salt, which may increase the solubility of the tissue-reactive material.

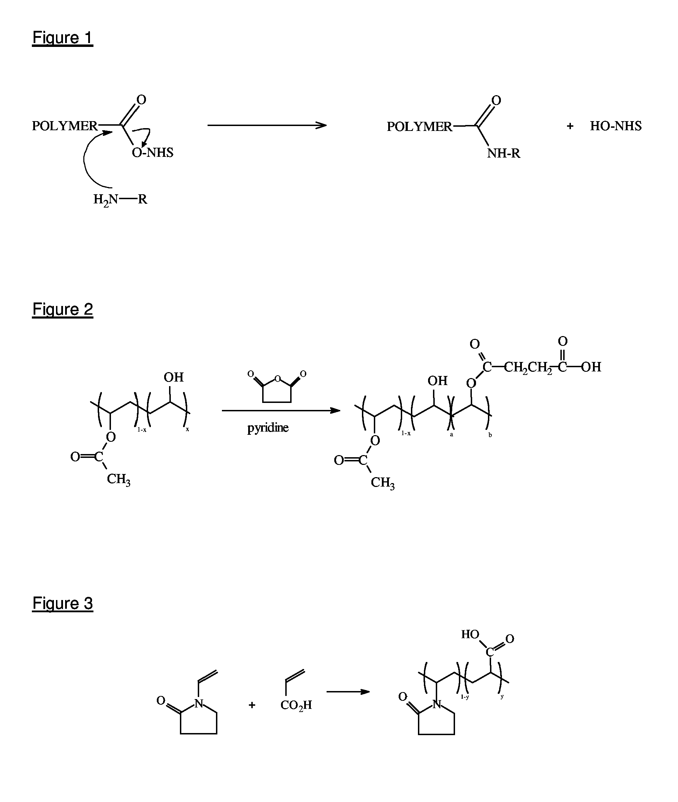

Figure 1 illustrates the mechanism by which an NHS-functionalised polymer reacts with an amine-containing material such as a tissue protein represented by R-NH₂. The reaction is a nucleophilic displacement leading to the formation of an amide bond between the polymer and the tissue protein.

Tissue-reactive functional groups that may be of utility in the present invention are any functional groups capable of reaction (under the conditions prevalent when the formulation is applied to tissue, ie in an aqueous environment and without the application of significant amounts of heat or other external energy) with functional groups present at the surface of the tissue. The latter class of functional group includes thiol and amine groups, and tissue-reactive functional groups therefore include groups reactive to thiol and/or amine groups. Examples are:
imido ester;
p-nitrophenyl carbonate;
NHS ester;
epoxide;
isocyanate;
acrylate;
vinyl sulfone;
orthopyridyl-disulfide;
maleimide;
aldehyde; and
iodoacetamide.

NHS ester is a particularly preferred tissue-reactive functional group.

In general, the tissue-reactive material may be formed by derivatisation of a suitable polymer precursor. Classes of polymer which lend themselves to such derivatisation include those that contain carboxylic acid or alcohol functional groups, or related structures. Polymers that may be used include polymers that are commercially available or polymers that are prepared specifically for this purpose. Naturally-occurring materials such as sucrose or a derivatised cellulose may also be used.

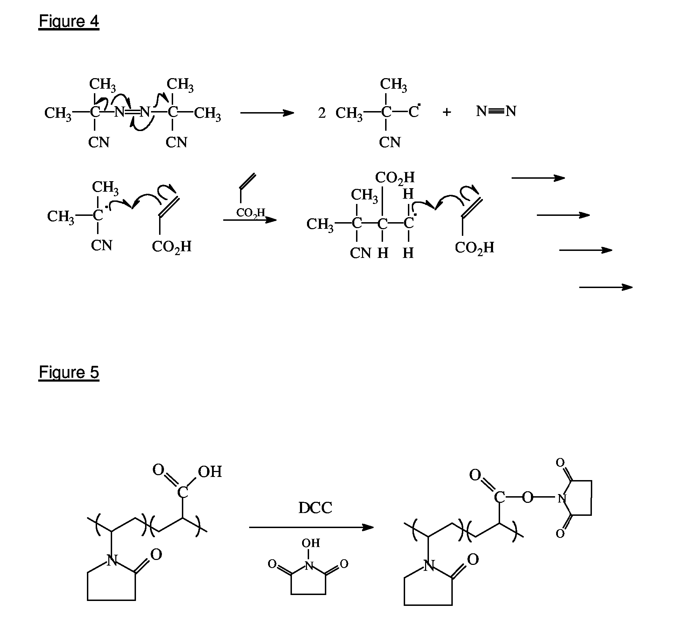

Commercially available polymers that may be used include PVA. In the case of PVA, the functional groups may be introduced by first adding a chain extending or linking group, for example an acid functionality that can be further reacted with NHS. Figure 2 shows the addition of a chain-extending group to a copolymer of vinyl acetate and vinyl alcohol, the chain-extending group terminating in a carboxylic acid group that may be converted to the corresponding NHS-ester. The copolymer starting material (in which molar fraction x of vinyl alcohol groups may be 0.85-0.995) is reacted with a cyclic anhydride (in the example illustrated, succinic anhydride) in the presence of a base such as pyridine. Between 5% and 40% of the alcohol groups are derivatised to form the carboxylic acid-bearing side chains (ie *a*+*b*=*x,* with a being between 0.05x and 0.40x), which may then be converted to the NHS-ester by conventional methods that are known *per se.*

Where the polymer support is synthesized for the purpose of subsequent derivatization, a wide variety of monomers may be used. Examples include *N*-vinyl-2-pyrrolidone, acrylic acid, vinyl acetate, vinyl acetic acid, mono-2-(methacryloyloxy)ethyl succinate, methacrylic acid, 2-hydroxyethyl methacrylate, 2-hydroxypropyl methacrylate, (polyethylene glycol) methacrylate or other monomers containing acid or alcohol functionality. Such monomers may be polymerised via various standard polymerisation techniques, including free radical techniques using an initiator such as benzoyl peroxide, AIBN, lauroyl peroxide, peracetic acid etc. One preferred example of such a polymer is poly(VP-AAc) polymerised using AIBN as initiator. The polymerization of this material is illustrated in Figure 3, in which the molar ratio of acrylic acid-derived units may be between 0 and 1.0, preferably less than 0.60, and more preferably less than 0.40, eg between 0.025 and 0.25. The copolymer may be further reacted with NHS to form the tissue-reactive material.

Preferably, all or substantially all of the available sites in the precursor to the tissue-reactive material will be derivatised (ie the tissue-reactive functional groups will be introduced into all or substantially all of the available sites in the precursor to the tissue-reactive material). The degree of binding between the tissue-reactive functional groups and the tissue to which the formulation is applied will then be a function of the amount of tissue-reactive material in the formulation.

Where, as is preferred, the tissue-reactive functional groups are NHS-esters, at least one of the monomers used in the preparation of the tissue-reactive material must contain a carboxylic acid group or a group capable of being reacted with another material to form an acid functionality.

ln the preferred case in which the tissue-reactive material is a derivative of poly(VP-AAc), the molar ratio of acrylic acid-derived units is preferably between 0.05 and 0.50, and hence that of the vinyl pyrrolidone-derived units is between 0.50 and 0.95. The derivative is referred to as activated poly(VP-AAc).

The acrylic acid groups are preferably derivatised to form tissue-reactive (activated) groups, most preferably with NHS groups. Poly(VP-AAc) in which the carboxyl groups of the acrylic acid-derived units carry NHS groups, is referred to herein as NHS-activated poly(VP-AAc).

The formulation may contain one type of tissue-reactive material, or more than one type of tissue-reactive material.

In addition to forming covalent bonds with the surface to which it is applied, the tissue-reactive material may also have bioadhesive properties. By this is meant that the material should exhibit good initial adhesion to biological tissue to which it is applied. Polymers with such properties typically contain chemical groups with a high ionic density, eg carboxyl, amide, lactam, hydroxyl; ether and ester groups, and the salts thereof, which interact cooperatively with tissue, through the formation of ionic and hydrogen bonds, dipole - dipole interactions and Van der Waals forces. Effective polymers are generally of high molecular weight since the degree of bioadhesion may be proportional to the number of these groups available. Typically, the molecular weight of a bioadhesive polymer will be in excess of about 50,000. The polymers are also generally linear, becoming physically entangled and having an amorphous distribution in solution.

For example, the tissue-reactive polymer may be derivatised PVP or a derivatised copolymer of vinyl pyrrolidone with another monomer (eg acrylic acid). In such a case, the pendant pyrrolidone groups will provide immediate contact adhesion (believed to be due to hydrogen and/or van der Waals bonding, as described above), while the tissue-reactive groups then form covalent bonds with functional groups within the tissue and within the matrix (cross-linking).

Sufficiency of the degree of initial adhesion of a sheet to the tissue can be quantitatively *determined in vitro,* for example by performing an adhesion strength test. This test is performed by allowing the sheet to adhere to a suitable substrate (secured in a fixed position), while the sheet itself is physically attached at a separate point to the load of a tensile testing apparatus, positioned so that, prior to the test, the sheet is not under load. The load cell is moveable along an axis substantially perpendicular to that along which the substrate is positioned. The test involves movement of the load cell away from the substrate, at a constant predetermined rate, until the sheet detaches from the substrate. The output of the test is a quantitative measure of the energy of adhesion for that sheet - ie the cumulative amount of energy required to break the interaction between the sheet and the substrate to which it is adhered. A suitable cumulative energy of adhesion for the sheet according to the invention would be not less than 0.1 mJ;: more preferably not less than 0.25mJ, and most preferably not less than 0.5mJ.

### Nature of the buffer material

The effect of the buffer is to enhance the reaction between the tissue-reactive material (which is typically an electrophile-rich material) and the surface of the tissue to which the formulation is applied (which typically contains nucleophilic groups).

It will be apparent to those skilled in the art that the reaction between electrophilic and nucleophilic compounds may be controlled by adjusting the pH of the reaction. As the pH is adjusted above 7, the reaction becomes more favourable. Thus, in preferred embodiments of the invention, the formulation is buffered to a pH greater than 7, more preferably greater than 9. The pH is preferably buffered to a pH in the range 8-12, more preferably 9-11.

Preferably, the buffer material consists entirely, or substantially entirely (eg more than 90% w/w, more preferably more than 95% w/w), of material that forms a buffer solution when hydrated, ie the buffer material preferably contains little or no other material that does not form part of a buffer system. Most preferably, the buffer material consists consists entirely, or substantially entirely (eg more than 90% w/w, more preferably more than 95% w/w), of inorganic material.

A wide range of buffer materials may be used, provided that they are biocompatible and of the correct pH. Examples include carbonate, phosphate, HEPES and borate buffers.

### Manufacture of the components of the formulation

The particulate tissue-reactive material and the particulate buffer material may be prepared by any suitable means. The buffer particles are preferably prepared by freeze-drying of an aqueous buffer solution. The tissue-reactive material is preferably isolated from an organic solution of the polymer.

The formulation may be prepared simply by admixing the components in particulate form, and where desired compacting the formulation to form tablets, plugs or the like. The degree of compaction should be such that the tablets etc retain their integrity until applied to tissue, but not so great as to inhibit hydration (and hence adhesion) after application.

### Physical forms of the formulation

The formulation according to the invention may have the form of a loose powder, in which particles of the tissue-reactive material are admixed with particles of the buffer material.

Alternatively, the formulation may take the form of a compacted body formed by compaction of the particles. Tissue-reactive materials based on PVP or copolymers of N-vinyl-2-pyrrolidone with other monomers (eg vinylic monomers) are particularly preferred in such applications, as PVP has suitable flow properties for blending with other components of the formulation, and exhibits excellent performance in dry granulation tableting processes as it undergoes plastic deformation on compression, and has low hygroscopicity.

ln a further alternative, the formulation may be applied to a support to form a sheet according to the invention.

Sheets in accordance with the invention may be planar, or may be folded, fluted, coiled or otherwise formed into more complex shapes.

The formulation may further comprise additional components such as structural polymers, surfactants, plasticizers and excipients commonly used in tablet manufacture. Such further components may be present as discrete particles, or may be components of the: particles of tissue-reactive material. ln preferred embodiments, however, the formulation is free, or substantially free, of such additional components. For instance, the formulation may comprise less than 2% w/w, more preferably less than 1 % w/w, or less than 0.5% w/w, of components other than the tissue-reactive material and the buffer material.

### Nature of the structural support

The principal functions of the support are to provide the sheet with structural integrity and to provide a flexible substrate onto which the tissue-reactive formulation, in powder form, can be applied. In most embodiments, the support is generally planar.

The support can be prepared using any suitable polymeric material or combination of materials. The support may be biodegradable or non-biodegradable, and should be biocompatible, ie should be capable of application to tissues either within or external to the body without causing any immunological or other adverse reaction. The support may comprise naturally occurring material, but is preferably free, or substantially free (eg less than 2% w/w, or less than 1% w/w or less than 0.5% w/w), of materials of human or animal origin, and is most preferably formed entirely of synthetic material.

### Examples of polymeric materials that may be used for the support are:

Polymers or co-polymers based on α-hydroxy acids, such as polylactide, polyglycolide, and also polycaprolactone and other polylactones such as butyro- and valerolactone.

Other examples may include:
alginates (ie polymers based on alginic acid, the polysaccharide obtained from seaweeds);
polyhydroxyalkanoates;
polyamides;
polyethylene;
propylene glycol;
water-soluble glass fibre;
starch;
cellulose;
collagen;
pericardium;
albumin;
polyester;
polyurethane;
polyetheretherketone;
polypropylene; and
polytetrafluoroethylene.

The support may be prepared by casting, spinning or foaming of a solution of the polymeric material, or by moulding, weaving of filamentous material, or slicing from a block of material. Appropriate techniques for the preparation of the support by such methods will be familiar to those skilled in the art.

In preferred embodiments, the support is formed with a regular array of apertures, for example in a square or hexagonal array. The apertures may be formed during manufacture of the support or may be introduced after the support is formed, for example by piercing.

Preferably, the apertures are between 50µm and 2mm in diameter and adjacent apertures are formed at a centre-to-centre separation of between 100µm and 5mm. Preferably, the apertures account for between 5% and 80% of the overall surface area of the support.

The support may have a thickness of from 0.005 to 5mm.

### Application of the formulation to the core

The formulation may be applied to just one side of the support. More preferably, however, the formulation is applied to both sides of the support.

Where the support is apertured, application of the formulation to one or both sides of the support causes the apertures to be filled with the formulation. In use, where the formulation is applied to both sides of an apertured support, the formulation that is present in the perforations effectively binds together the activated coatings on each side of the support, encapsulating the support between the two layers of activated coating.

The preferred method for applying the formulation to the support involves mechanically compressing (eg using a hydraulic press) a blend of the tissue-reactive material and the buffer material, both in particulate form, onto one or both sides of the support.

The blend may be prepared by admixing particles of the tissue-reactive material with particles of the buffer material.

The thickness of the coating applied to one or both sides of the support will typically be between 50pm and 500µm, more commonly from about 70µm to about 200µm.

Optionally, a surface of the sheet that, in use, is not intended to adhere to tissue may be coated with a non-adhesive material. Most preferably, such a material is a synthetic polymer. Examples of suitable polymers include polyethylene glycols, polylactide and PLG. A sheet with such a non-adhesive coating will adhere only to the target tissue (to which the underside of the sheet is applied) and not to surrounding tissues (eg the pleural or peritoneal wall). The non-adhesive coating may include a visibly-absorbing chromophore to enable identification of the non-tissue contacting surface of the sheet. An example of a suitable chromophore is methylthioninium chloride.

The non-adhesive coating is preferably also formed with apertures. ln such a case, the apertures may be formed in a similar array to the apertures in the core, with similar separations between apertures. The apertures in the non-adhesive coating are, however, preferably somewhat smaller than those in the core, eg with a diameter of between 50pm and 1mm.

### Physical form of the sheet

The sheet may typically have an overall thickness of from 0.05 to 10 mm, typically 0.05 to 2mm, and more commonly 0.05 to 0.5 mm, eg about 200µm or 300pm or 400µm.

The sheet may be produced with, or subsequently cut to, dimensions of from a few square millimetres up to several tens of square centimetres, or more.

### Therapeutic applications of the formulation and sheet

The formulation and sheet according to the invention are suitable for application to both internal and external surfaces of the body, ie they may be applied topically to the exterior of the body (eg to the skin) or to internal surfaces such as surfaces of internal organs exposed during surgical procedures, including conventional and minimally invasive surgery.

The formulation and sheet according to the invention are particularly suitable for surgical applications in the following areas:
Thoracic / cardiovascular
General surgery
ENT
Urology
Oral / maxillofacial
Orthopaedic
Neurological
Gastroenterology
Ophthalmology
Gynaecology / obstetrics
Possible uses are described in more detail below.

### Wound healing

The degradable nature of the formulation and sheet mean that they may support and promote wound healing during both internal and topical procedures. Once the formulation and/or sheet begin to degrade, fibroblasts will move in and begin to deposit components of the extracellular matrix. The formulation and sheet can therefore be used as an internal or external dressing. In addition, factors such as growth factors and cAMP that are known to promote the proliferation of skin cells may be added to the formulation to assist in the healing process. The sheet may be designed to control the transmission of moisture and infectious agents, and thus be useful particularly in the treatment of burns.

### Skin closure

The formulation and sheet may be applied topically to promote wound closure (as an alternative to sutures). This may have beneficial effects in that it may reduce scarring, and the formulation and sheet may thus be useful for cosmetic purposes during minor surgery (eg in Accident & Emergency Departments). Self-adhesive properties of the sheet may make it easy to apply quickly.

### Hernia repair

The sheet may be used to provide reinforcement in hernia repair procedures. The self-adhesive attachment overcomes the potential issues faced by conventional surgical reinforcing mesh products, which require suturing or stapling in an already weakened area. The sheet for such a procedure may be engineered to have short or long term durability, depending on the degree of tissue repair required. The sheet may also be able to withstand the application of staples.

### Anastomosis

The formulation and self-adhesive sheet provide a means for rapid sealing of, and prevention of leaks in, joined tubular structures such as blood vessels, and vascular and bladder grafts, and the Gl tract. The ability of the sheet to support tissue repair may be of particular value if used in nerve repair.

### Sealing large areas of tissue

The good sealing and handling properties of the formulation and sheet, combined with their self-adhesive properties and ability to cover a large surface area, mean that they may be of particular use in sealing resected tissue surfaces - in particular those where diffuse bleeding is an issue (eg the liver). The sheet also provides an ideal support matrix for tissue repair at such sites. This could also be applicable to limiting leakage of cerebro-spinal fluid following neurological surgery.

### Sealing air leaks

In addition to the patch properties described above, the high tensile strength and good inherent elasticity of the formulation and sheet (after hydration and reaction of the tissue-reactive functional groups), make them particularly suitable for sealing air leaks in the lung, particularly following lung resection. Again, after effecting a seal, the sheet provides an ideal support matrix for tissue repair at such sites.

### Haemostasis

The formulation and sheet may be applied to a bleeding area, acting as a physical barrier. The tissue-reactive material in the formulation and sheet may immobilise proteins and thereby promote haemostasis.

### Therapeutic agent administration

Drugs and other therapeutic agents (including biologically active agents such as growth factors, and even cells and cellular components) may be added to solution(s) used to form the components of the formulation and sheet, or covalently linked to components prior to their use in the manufacture of the formulation and sheet, or simply admixed in particulate form with the tissue-reactive material and particulate buffer material. Once the formulation or sheet is in place, following application to the desired site, the drug will be slowly released, either by diffusion or by engineering the formulation or sheet so that as it degrades over time the drug is released. The rate of release can be controlled by appropriate design of the formulation and sheet. The formulation and sheet may thus provide a means for delivering a known amount of drug either systemically or to a precise locus. The drug may be directly bound to a component of the formulation, or simply dispersed in the formulation.

### Prevention of Post-Surgical Adhesions

Post-surgical adhesion, the formation of undesired connective tissue between adjacent tissues, is a serious problem which can give rise to major post-surgical complications. It is a particular problem in bowel surgery where it can cause, for instance, twisting of the bowel, which may then necessitate further surgical intervention. The application of sheet material having self-adhesive properties in accordance with the invention to tissues exposed in a surgical procedure can be effective in preventing post-surgical adhesions between that tissue and neighbouring tissues.

### Minimally Invasive Procedures

The use of minimally invasive techniques for taking tissue samples by biopsy, inserting devices, delivery of therapeutic agents and performing surgical procedures is rapidly developing as an alternative choice to traditional "open" surgery. Minimally invasive procedures typically result in less pain, scarring, quicker recovery time and fewer post-operative complications for patients, as well as a reduction in health care costs. Procedures are undertaken using specially designed instruments which are inserted through small keyhole-sized surgical incisions. The formulation and sheet may be introduced into the body via existing and specially designed minimally invasive surgery instruments and trocar systems, and the sheet may be shaped or prepared to an appropriate size and configuration. The format of the formulation also may be modified to enable delivery of powders, tablets, pellets, tapes/strips/plegets and other 3-D matrices. The use of a self adhesive formulation will significantly reduce the technical difficulties associated with manipulating, closing and repairing tissues where access is restricted. ln addition the sheet properties make them particularly suitable for sealing leaks of air, blood or fluid or for delivery of therapeutic agents.

### Detailed Description of Preferred Embodiments

The invention will now be described in greater detail, by way of illustration only, with reference to the following Examples.

### Example 1

### Synthesis of poly(VP-AAc(NHS))

### 1.1 Polymerisation of acrylic acid-land N-vinyl-2-pyrrolidone

The polymer is formed via the polymerisation of monomers such as N-vinyl-2-pyrrolidone and acrylic acid, as shown in Figure 3.

A number of methods may be used to initiate the polymerisation, such as free radical, ionic (cationic or anionic), thermal, UV, redox etc. Free radical polymerisation is the preferred polymerisation method and AIBN is the preferred initiator. The AIBN decomposes into two radicals which can then attack the carbon-carbon double bond in the vinylic monomer (acrylic acid) as shown in Figure 4.

This will continue until termination of chain growth, via combination, disproportionation etc.

The reaction solvent may be DMF, toluene, or any other suitable solvent with a boiling point greater than 100°C. Toluene is the currently preferred solvent.

### A typical polymerisation method is as follows:

Solvent is charged to the reaction flask. Usually, around 5-10 ml of solvent per gram of monomer is sufficient. The flask is heated in an oil bath to a temperature sufficient for the generation of free radicals from the chosen initiator. 80-85°C is the optimum temperature when using AIBN as the initiator. Oxygen-free nitrogen is bubbled through the solvent to remove any dissolved oxygen. Oxygen is also removed from the monomers in the same manner. The initiator is added to the solvent and allowed to dissolve. The monomers are added and the vessel closed. A nitrogen inlet and an escape needle may also be used.

The reaction may be allowed to stand for around 3-24 hours. The reaction mixture is cooled and the polymer is isolated from the solvent/polymer solution by precipitation in 5:1 hexane/isopropanol followed by filtration. Successive washes with diethyl ether are required to remove all traces of polymerisation solvent from the polymer. After successive diethyl ether washes, the polymer is dried under reduced pressure to constant weight.

Typical reaction conditions are shown in Table I:

**Table I**

| Solvent (vol) | Monomer (g) | | AIBN (g) | T (°C) | Time (hrs) | Yield | Mw | Mn | Polydispersity Index (Mw/Mn) |
|---|---|---|---|---|---|---|---|---|---|
| | acrylic acid | N-vinyl-2- pyrrolidone | | | | | | | |
| Toluene (100ml) | 1.5 (20mol%) | 8.5 (80mol%) | 0.02 (0.125%) | 80 | 3 | - | - | - | - |
| Toluene (100ml) | 0.7 (10mol%) | 9.3 (90mol%) | 0.02 (0.0125%) | 80 | 3 | 54% | 80040 | 38800 | 2.0 |
| Toluene (100ml) | 0.7 (10mol%) | 9.3 (90mol%) | 0.04 (0.25%) | 80 | 3 | 58% | 74240 | 38340 | 1.9 |
| DMF (100ml) | 0.7 (10mol%) | 9.3 (90mol%) | 0.02 (0.125%) | 80 | 3 | 62% | 54000 | 25150 | 2.1 |
| Toluene (100ml) | 0.5 (7.5mol%) | 9.5 (92.5mol%) | 0.02 (0.125%) | 80 | 3 | - | - | - | - |
| Toluene (100ml) | 0.35 (5mol%) | 9.65 (95mol%) | 0.02 (0.125%) | 80 | 3 | - | - | - | - |

### 1.2 Reaction of poly(VP-AAc) and NHS in the presence of DCC

Poly(VP-AAc(NHS)) is formed from the reaction of poly(VP-AAc) and NHS in the presence of DCC (Figure 5).

10g of poly(VP-AAc) containing 0.094 moles of acrylic acid repeat units is dissolved in 50 ml of dried DMF by stirring in a dry 100ml round bottomed flask. 0.01 moles of NHS (1.15g) is added to the polymer solution and is allowed to dissolve.

DCC (2.06g) is melted in an oven at 60°C and added to the polymer solution. This is left to stir at room temperature for at least 24 hours. The formation of a white precipitate (DCU) is observed. After 24 hours the precipitate is removed by filtration, and the flask and filter washed with a small amount of dry DMF. The polymer is isolated by precipitation in 5:1 hexane/iso-propanol and filtration. The polymer is further purified by repeated washes with dry diethyl ether. The yield is between 50% and 70%.

### Example 2

### Alternative synthesis of poly(VP-AAc(NHS))

### 2.1 Polymerisation

400ml of dried toluene is heated to 80±2°C in a round bottomed flask using an oil bath or isomantle. Oxygen is removed from the solvent by bubbling oxygen-free nitrogen through the toluene for at least 30 minutes. 0.1g (0.006 moles) of AIBN dissolved in 2ml of toluene is added to the reaction flask using a syringe, immediately followed by 45.02g (0.406 moles) of 1-vinyl-2-pyrrolidone and 7.02g (0.092 moles) of acrylic acid. The reaction is left under nitrogen at 80±2°C for 17 hours; the polymer is insoluble in toluene and forms a white precipitate as the reaction proceeds. After 17 hours, a further 0.1 g (0.006 moles) of AIBN is added and the reaction is kept at 80±2°C for one further hour to polymerise any remaining monomer. The polymer is isolated by pouring into 2000 ml of rapidly stirred 1:1 hexane:diethyl ether and subsequent filtration using a 10-16µm filter. The polymer is dissolved in 200ml of DMF and stirred for approximately 60 minutes before being filtered through a 10-16µm filter. The polymer is precipitated in approximately 2000ml of rapidly stirred 5:1 hexane:iso-propanol and isolated by filtration using a 10-16µm filter. All traces of DMF and toluene are removed by washing and filtration with 500ml of diethyl ether three times. The polymer is dried for at least 72 hours at 60 °C *in vacuo.*

### 2.2 NHS-esterification.

The acid content of the polymer is calculated by titration against 1.0M NaOH.

20g of the polymer are dissolved in 160ml of dry DMF in a 250ml round-bottomed flask using a magnetic stirrer. 4.28g (0.037 moles) of NHS are added and allowed to dissolve. 7.67g (0.037 moles) of DCC are dissolved in 10ml of dry DMF and added to the polymer/NHS solution. The flask is sealed and the reaction stirred for 96 hours at room temperature. DCU is formed as a reaction by-product and this is apparent as a white precipitate present in the reaction solution. After 96 hours the DCU is removed by filtration using a 10-16µm filter and the polymer isolated by precipitation using 1275ml of 5:1 hexane:iso-propanol. This is removed by filtration using a 10-16µm filter. The polymer is purified further by dissolving in 170ml of DMF and precipitation in 1275ml of 5:1 hexane:iso-propanol three further times. After the final precipitation the polymer is washed by stirring rapidly in 170ml of diethyl ether until a fine white powder is obtained. This is dried for at least 72 hours at 60°C *in vacuo.*

### Example 3

### Further alternative synthesis of poly(VP-AAc(NHS))

### 3.1 Polymerisation

600ml of dried toluene is heated to 80±2°C in a round bottomed flask using an oil bath or isomantle. Oxygen is removed from the solvent by bubbling oxygen-free nitrogen through the toluene for at least 30 minutes. 0.144g (8.8 x 10⁻⁴ moles) of AIBN dissolved in 3ml of toluene is added to the reaction flask using a needle and syringe, immediately followed by 64.88g (0.576 moles) of 1-vinyl-2-pyrrolidone and 10.11 g (0.140 moles) of acrylic acid. The reaction is left under nitrogen at 80±2°C for 17-19 hours; the polymer is insoluble in toluene and forms a white precipitate as the reaction proceeds. After 18±1 hours, the polymer is isolated by pouring into 2880ml of rapidly stirred 1:1 hexane:diethyl ether and subsequent filtration under reduced pressure using a 10-16µm filter. The polymer is purified further by three successive washes with 600ml of diethyl ether, each wash being followed by filtration under reduced pressure using a 10-16µm filter. The polymer is dried for at least 72 hours at 40°C *in vacuo.*

### 3.2 NHS-esterification.

The acid content of the polymer is calculated by titration against 1.0M NaOH.

55g of the polymer prepared in Example 3.2 is dissolved in 460ml of dry DMF in a 1 000ml round-bottomed flask using a magnetic stirrer. 12.74g (0.11 moles) of NHS are added and allowed to dissolve. 22.81 g (0.11 moles) of DCC is dissolved in 46ml of dry DMF and added to the polymer/NHS solution. The flask is sealed and the reaction stirred for 96 hours at room temperature. DCU is formed as a reaction by-product and this is apparent as a white precipitate present in the reaction solution. After 96 hours the DCU is removed by filtration under reduced pressure using a 10-16µm filter and the polymer isolated by precipitation using 3436ml of rapidly stirred 5:1 hexane:iso-propanol. After the precipitation the polymer is washed by three successive washes using 600ml of diethyl ether. This is dried for at least 72 hours at 60°C *in vacuo.*

### 3.3 Purification by Soxhlet extraction

Two Soxhlet extraction thimbles, each containing 25g of the polymer prepared above are loaded into a Soxhlet extraction apparatus. 800ml of iso-propanol is charged into a 1000ml round bottomed and heated to reflux. The extraction is run for one hour, the solvent is changed and the extraction is run for a further three hours before the solvent is changed once more. The extraction is then run for a further hour before being terminated. The polymer is then washed with 900ml of diethyl ether twice and then dried at 40°C *in-vacuo* for at least 16 hours.

### 3.4 Density optimisation

Subsequent to the purification of the polymer by Soxhlet extraction the appearance of the polymer is improved by dissolving in a solvent mixture to remove any hard lumps formed. 50g of PVP(NHS) is dissolved in 425ml of 15/4 v/v DCM/methanol and then subsequently precipitated from 2500ml of diethyl ether. The polymer is isolated by filtration under reduced pressure using a 10-16µm filter and then washed twice with 1000ml of diethyl ether. The polymer is then dried at 40°C *in-vacuo* for at least 72 hours.

### Example 4

### Preparation of particulate buffer material

5.72g of sodium phosphate monobasic and 26.65g of sodium carbonate was made up to 1000ml using water for injections. The solution was freeze-dried to give a powder.

### Example 5

### Blending of poly(VP-AAc(NHS)) with buffer material

Powdered poly(VP-AAc(NHS)), prepared as in Example 1, is blended with buffer material, prepared as in Example 4, in a ratio of 95:5 by weight.

### Example 6

### Schematic representation of a sheet according to the invention

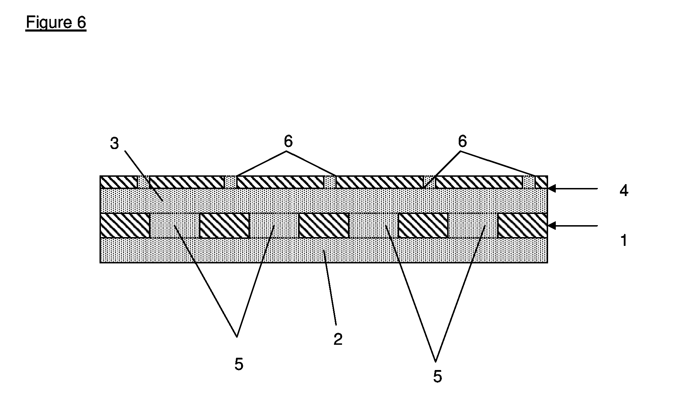

Figure 6 shows (schematically and not to scale) the structure of a typical sheet prepared in accordance with the invention. The sheet comprises a core in the form of a film 1 of PLG which has a regular array of apertures 5. Layers 2,3 of a tissue-reactive formulation are pressed onto both sides of the film 1 such that the tissue-reactive formulation penetrates into, and fills, the apertures. Finally, a non-adhesive layer 4, again of PLG, is applied to one surface of the sheet. The non-adhesive layer 4 is also perforated, the perforations 6 being smaller than the apertures 5 in the core film 1.

The non-adhesive layer 4 may include a chromophore that gives the non-adhesive surface a discernible colour, thereby identifying that surface (and hence indicating which side of the sheet is to be applied to the tissue). Alternatively, the two sides of the sheet may be distinguishable by virtue of a difference in reflectivity of the two surfaces.

### Example 7

### Preparation of multilayer sheet formulation

This Example describes the preparation of a multilayer tissue-adhesive sheet having the structure described in Example 6. The sheet comprises a PLG core, to both sides of which a particulate mixture of poly(VP-AAc(NHS)) and buffer material is applied. A PLG barrier layer is applied to one side of the sheet.

### 7.1 Preparation of PLG core film

The core film is prepared by casting from a 10 % w/w solution of PLG in DCM. The core film is produced by spreading the 10 % polymer solution using a 300µm K Bar (R K Print Coat Instruments Ltd, Royston, UK) on silicon paper. A K Bar is a device for accurately producing films of a specific thickness from a specific concentration solution. After drying, the thickness of the core film is 30µm.

### 7.2 Preparation of PLG barrier film

The barrier film is produced in an analogous manner to the core film, but using a 24µm K Bar. After drying, the thickness of the barrier film is 3µm.

### 7.3 Preparation of perforations and cutting out of PLG core and barrier films

The next step is to perforate the core and barrier films. This is carried out using a heated perforating device in the form of a press adapted for the purpose. The press is fitted with a heated plate, the underside of which is formed with a regular array of pyramidal projections.

The perforations are created by applying the heated plate with pressure to the film. This softens the polymer film, which redistributes itself around the small pyramids. For perforating the core film, the heated plate is set to 90ºC and pressed for 10s. The barrier film is perforated using a temperature of 90ºC for 5s at a reduced pressing pressure. The perforation size for the core film is 1.3 mm with centre separations of 2.5 mm and the barrier film perforation size is 0.5 mm with centre separations of 2.5 mm. The perforated films are then cut to size, eg a circle of 39.8mm diameter. Due to redistribution of the film material during perforation, the film thickness increases to around 80µm for the core film and around 10µm for the barrier film.

### 7.4 Preparation of poly(VP-AAc(NHS))

Poly(VP-AAc(NHS)) was prepared as described in Example 1.

### 7.5 Preparation of freeze-dried buffer

Freeze-dried buffer was prepared as described in Example 4.

### 7.6 Preparation of mixed poly(VP-AAc(NHS)) / buffer powders

The poly(VP-AAc(NHS)) and buffer material are ground together in a pestle and mortar until a fine powder is obtained. The ground powder is then mixed on a roller mixer for 30min prior to use to ensure the two components are fully integrated.

### 7.7 Preparation of multilayer sheet

The final product is assembled in a Specac FT-IR 40mm die by compression between two pellets. Pieces of silicon paper are used to prevent the finished product sticking to the pellets.

With the first pellet in the die, a silicon paper disc is placed in the cavity and a 150mg portion of the ground powder is sprinkled onto the silicon paper. The powder is carefully manipulated with a spatula or plunger so that the powder evenly covers the entire base of the die. A 39.8mm diameter perforated core film is placed on top of the powder layer and firmly pressed so that so that the film is flat and in contact with the powder layer beneath it so that the powder occupies the perforations of the core film. A second 150mg aliquot of powder is sprinkled onto the perforated PLG core film and again gently levelled.

The perforated barrier layer is placed on a second piece of silicon paper and positioned on top of the second powder layer.

A second die pellet is introduced, and the assembled die placed into the press and compressed to a pressure of 2 tonnes for 30s. The final product is then removed from between the pellets. The thickness of the final product is in the range 325-425µm.

The side of the sheet to which the barrier layer is applied has a shiny appearance, and is hence distinguishable from the matt surface of the tissue-reactive side.

### Example 8

### Application of sheet to tissue

The tissue surface is prepared in accordance with conventional surgical techniques. The sheet is applied onto (and if necessary) around the tissue surface with moderate pressure for a period of 30 seconds to ensure satisfactory contact to the tissue.

### Example 9

### Measurement of adhesive strength

A Universal testing machine (UTM, Zwick/Roell BZ2,5) may be used to test the adhesive strength of test materials to freshly excised liver or lung tissue. Details of the testing procedure are summarised below.

A small section of tissue (4 cm x 4 cm x 1 cm (depth)) is prepared and mounted into a purpose-made holder at the base of the test machine. The surface of the tissue was sprayed with water. The test specimen (with sample holder attached to enable subsequent removal) is placed onto the tissue surface with a moderate force to ensure full contact. The material is left on the tissue for 5 minutes and then wholly submerged in water for a further 5 minutes. Whilst holding the tissue in place using a suitable clamp the folded tip of the sample holder is inserted in the grips of the UTM. The sample is positioned appropriately to ensure that the sample is aligned with the grips. The grip is then moved at 90° from the test sample thereby removing the sample from the tissue. The UTM software (Zwick TestXpert ver 9.0) can be used to calculate the energy of adhesion (mJ) of the test material.

## Claims

1. A tissue-adhesive formulation comprising a particulate material having tissue-reactive functional groups, in admixture with a particulate buffer material.

2. A tissue-adhesive formulation as claimed in Claim 1, which is free or substantially free of materials of human or animal origin.

3. A tissue-adhesive formulation as claimed in Claim 1 or Claim 2, that consists, or consists essentially of, an anhydrous or partially hydrated blend of particulate material having tissue-reactive functional groups and particulate buffer material.

4. A tissue-adhesive formulation as claimed in any preceding claim, wherein the ratio by weight of tissue-reactive material to buffer material is between 90:10 and 99:1, more preferably between 92:8 and 97:3.

5. A tissue-adhesive formulation as claimed in any preceding claim, wherein the tissue-reactive groups are NHS ester groups.

6. A tissue-adhesive formulation as claimed in any preceding claim, wherein the formulation is buffered to a pH in the range 8-12, more preferably 9-11.

7. A tissue-adhesive formulation as claimed in any preceding claim, wherein the buffer material comprises carbonate, phosphate, HEPES or borate buffers.

8. A sheet having a multilayer structure, said structure comprising a structural support coated on at least one side thereof with a tissue-adhesive formulation as claimed in any preceding claim.

9. A method of joining a tissue surface to another tissue, or of sealing a tissue surface, which method comprises applying to the tissue surface a formulation as claimed in any one of Claims 1 to 7 or a sheet as claimed in Claim 8.
